Europäisches Patentamt

European Patent Office

Office européen des brevets

(19)

(11) Veröffentlichungsnummer: **0 102 564**

**A2**

(12) # EUROPÄISCHE PATENTANMELDUNG

(21) Anmeldenummer: 83108078.3

(22) Anmeldetag: 16.08.83

(51) Int. Cl.³: **A 61 M 1/03**
**A 61 K 35/14**

(30) Priorität: 17.08.82 DE 3230540

(43) Veröffentlichungstag der Anmeldung:
14.03.84 Patentblatt 84/11

(84) Benannte Vertragsstaaten:
DE FR IT

(71) Anmelder: Fraunhofer-Gesellschaft zur Förderung der
angewandten Forschung e.V.
Leonrodstrasse 54
D-8000 München 19(DE)

(72) Erfinder: Chmiel, Horst, Prof. Dr.-Ing.
Paracelsusstrasse 14
D-7250 Leonberg-Ramtel(DE)

(72) Erfinder: Kern, Peter, Dipl.-Ing.
Laurentius-Strasse 24
D-7140 Ludwigsburg(DE)

(72) Erfinder: Kulbe, Klaus Dieter, Dr.
Ritterstrasse 12
D-7031 Gärtringen 2(DE)

(72) Erfinder: Hellwig, Günther, Dr.
Im Langen Hau 2
D-7000 Stuttgart 80(DE)

(74) Vertreter: Kraus, Walter, Dr. et al,
Patentanwälte Dres. Kraus & Weisert Irmgardstrasse 15
D-8000 München 71(DE)

(54) Verfahren zur Entfernung von protein- oder lipidgebundenen Toxinen aus Blut oder Blutfraktionen und die Verwendung bestimmter Verbindungen als Entkopplersubstanzen.

(57) Die Erfindung betrifft ein Verfahren zur Entfernung protein- oder lipidgebundener Toxine, insbesondere von Thiolen, aus dem Blut oder aus Blutfraktionen bei welchem zur Trennung des Protein- Toxin- oder Lipid-Toxin-Komplexes Verdränger- substanzen im Überschuß verwendet werden, die eine höhere Affinität zum Protein bzw. Lipid als das betreffende Toxin besitzen oder das Toxin durch reversible Auffaltung der Proteinstruktur freisetzen.

Sie betrifft weiterhin ein Verfahren zur Entfernung der freigesetzten Toxine durch adsorptive, chemische oder physikalische Bindung an einen neuen Partner (Fängersubstanz), oder durch Membrantrennprozesse oder durch enzymatische Umwandlung, sowie dessen Verwendung.

Verfahren zur Entfernung von protein-oder lipidgebundenen Toxinen aus Blut oder Blutfraktionen
und die Verwendung bestimmter Verbindungen als
Entkopplersubstanzen

---

Die Erfindung betrifft ein Verfahren zur Entfernung von
protein- oder lipidgebundenen Toxinen aus Blut oder Blutfraktionen und die Verwendung bestimmter Verbindungen als
Entkopplersubstanzen.

Nach neueren Erhebungen treten in der Bundesrepublik jährlich etwa 80 000 Vergiftungsfälle auf, wovon etwa 6 000
einen tötlichen Ausgang haben. Dabei ist bei Kindern die
Hauptursache in der Einnahme unbeaufsichtigt erreichbarer
giftiger Stoffe, wie Arznei-, Pflanzenschutz-, Schädlingsbekämpfungs- und Reinigungsmittel, zu sehen, während bei
Erwachsenen meist Suizidabsichten zugrundeliegen.

Weitere Vergiftungen treten bei Menschen mit akutem oder
chronischem Leberversagen auf.Zytostatika müssen oft über
längere Zeit in hohen Dosen verabreicht werden, sammeln
sich daher im menschlichen Körper an und üben dann toxische
Wirkungen aus.

In der medizinischen Praxis stellt sich somit häufig die
Aufgabe, Substanzen aus dem Körper zu entfernen, die eine
toxische, für den Patienten lebensgefährliche Konzentration erreicht haben. Diese Substanzen werden im folgenden
als Toxine bezeichnet.

Dabei ist zu beachten, daß diese Substanzen im Blut teils
ungebunden, teils an Serumproteine oder an Lipide gebunden
vorliegen. Die Lage des Gleichgewichts hängt in erster Linie vom Toxin selbst ab. Daraus folgt, daß die Beseitigung von überwiegend protein- oder lipidgebundenen Toxinen durch Entfernung des freien ungebundenen Anteils (Verschiebung des chemischen Gleichgewichts) ein langwieriges Verfahren darstellt und bei akuten Vergiftungen häufig nicht rasch genug zum Ziel führt. Gerade bei akuten Vergiftungen ist es erforderlich, schnell Maßnahmen in die Wege zu leiten, um das Leben des Patienten zu retten.

Die bisherigen Verfahren zur Entfernung von Toxinen mit starker Bindung an Proteine oder Lipide waren sehr langwierig, da nur der ungebunden frei vorliegende Teil an Toxin entfernt werden konnte und für eine weitergehende Entfernung zunächst die Nachstellung des Gleichgewichts abgewartet werden muß.

In der DE-OS 30 04 990 wird eine Vorrichtung zur Entgiftung von Blut und Blutkompartimenten beschrieben. Bei dieser bekannten Vorrichtung wird das Blut längs einer semipermeablen Membran entlanggeströmt, wobei das mit Toxinen beladene Fluid durch die Poren der Membran durchtritt. Auf der dem Blut bzw. dem Blutkompartiment abgekehrten Seite der semipermeablen Membran ist ein Depot vorgesehen, das mindestens einen Akkregationshemmer und mindestens einen Katalysator enthält. Die Membran ist für das mit Toxinen beladene Fluid auf der einen Seite und für den Aggregationshemmer und den Katalysator auf der anderen Seite durchlässig. In dieser Offenlegungsschrift wird jedoch nicht erwähnt, welche Katalysatoren für die verschiedenen Toxine geeignet sind. Ohne derartige Katalysatoren ist es jedoch bei der bekannten Vorrichtung nicht möglich, viele Toxine aus dem Blut zu entfernen.

In der DE-OS 26 42 535 wird ein Blutbehandlungssystem beschrieben, bei dem das Blutplasma mit Hilfe einer Membran abgetrennt wird und das Blutplasma gereinigt wird. Bei dieser bekannten Vorrichtung erfolgt die Reinigung mit Aktivkohle.

Es ist jedoch ein Nachteil von Aktivkohle, daß sie nicht vollständig aus dem Plasma entfernt werden kann und daher immer geringe Mengen an Aktivkohle noch in dem Blutplasma vorhanden sind.

Der vorliegenden Erfindung liegt somit die Aufgabe zugrunde, ein Verfahren und die Verwendung bestimmter Verbindungen zur schnellen Entfernung von protein- oder lipidgebundenen Toxinen zur Verfügung zu stellen. Das Verfahren und die verwendeten Verbindungen sollen insbesondere bei akutem Leberversagen, bei Suizidversuchen und bei der Verabreichung von Zytostatika oder anderen toxischen Medikamenten Anwendung finden.

Gegenstand der Erfindung ist ein Verfahren zur Entfernung von protein- oder lipidgebundenen Toxinen aus Blut oder Blutfraktionen, das dadurch gekennzeichnet ist, daß man zu dem Blut oder der Blutfraktion Entkopplersubstanzen zugibt, welche die Toxin-Protein-Bindung oder Toxin-Lipid-Bindung lösen, und die freigesetzten Toxine aus dem Blut oder der Blutfraktion entfernt.

Bei einer bevorzugten erfindungsgemässen Ausführungsform des Verfahrens trennt man vom Blut außerhalb des Körpers die Fraktion ab, die die protein- oder lipidgebundenen Toxine enthält , aus dieser Fraktion werden die Toxine entfernt und die von Toxinen befreite Blutfraktion wird dann mit den anderen Blutbestandteilen wieder vereinigt.

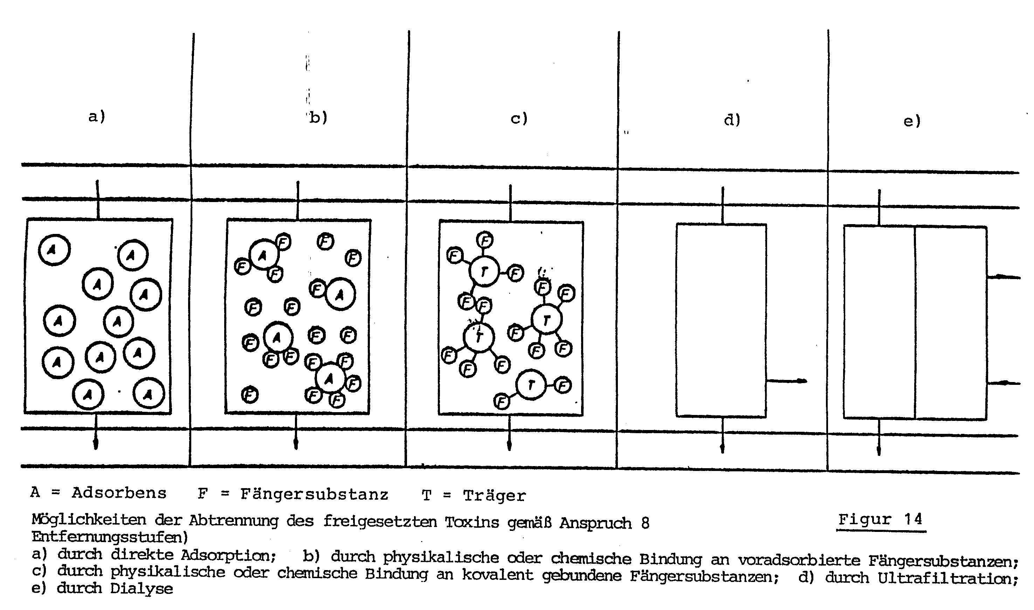

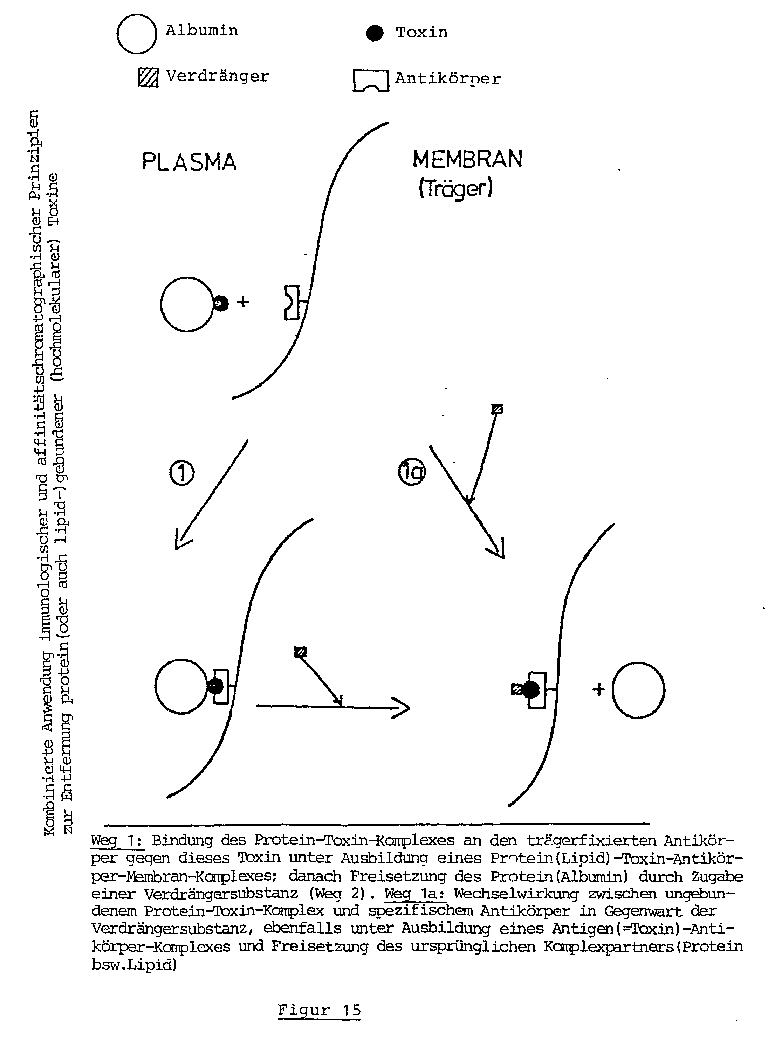

Bei einer weiteren bevorzugten Ausführungsform des erfindungsgemäßen Verfahrens werden die Toxine nach ihrer Freisetzung durch die Entkopplersubstanz aus dem Blut oder der Blutfraktion durch Adsorption, chemische oder physikalische Bindung an einen neuen Partner (Fängersubstanz) durch Membrantrennprozesse und/oder enzymatische Umwandlung,wie es beispielsweise in den Fig. 14 und 15 dargestellt ist, entfernt.

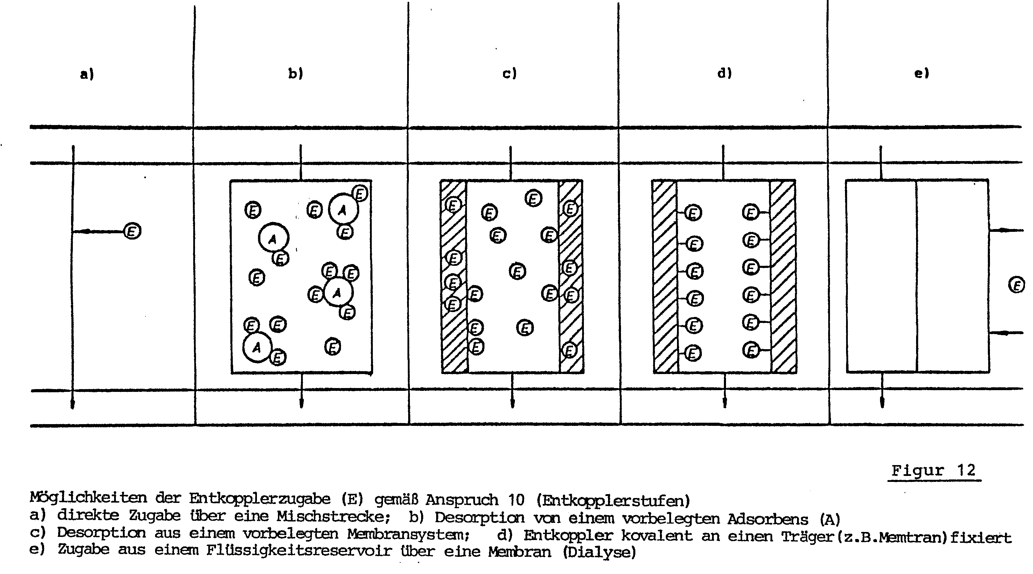

Gemäß einer weiteren bevorzugten Ausführungsform des erfindungsgemäßen Verfahrens werden die entkoppelnden Substanzen in der Entkopplereinrichtung in freier gelöster Form direkt über eine Mischstrecke oder desorptiv aus einem vorbelegten Adsorbens oder aus einem vorbelegten Membransystem oder aus einem Flüssigkeitsreservoir mittels eines Membranprozesses zu der Plasmafraktion oder zum Plasma zugegeben oder sie sind in kovalenter Bindung an einer Membran vorhanden, wie es beispielsweise in Figur 12 dargestellt ist.

Gemäß einer weiteren bevorzugten erfindungsgemäßen Ausführungsform enthält die Toxinentfernungsstufe Fängersubstanzen, die adsorptiv oder kovalent an einem Träger vorliegen.

Bei einer weiteren bevorzugten erfindungsgemäßen Ausführungsform des Verfahrens wird die Entkopplereinrichtung und/oder die Toxinentfernungseinrichtung nur einmal oder rezirkulierend durchströmt.

Die Erfindung betrifft weiterhin die Verwendung von physiologischen oder nicht physiologischen Verbindungen und/oder ihren Gemischen als Entkopplersubstanzen, wobei diese Verbindungen die dem zu verdrängenden Toxin entsprechende chemische Strukturelemente, wie funktionelle Gruppen, und physikochemische Eigenschaften, wie Hydrophilie- bzw. einen Hydrophobizitätsgrad, aufweisen und/oder in der Lage sind, das jeweilige Toxin chemisch oder physikalisch zu binden (Fängersubstanzen) oder die Tertiärstruktur des Toxinträgers (Protein, Lipid) in reversibler Weise aufzuheben.

Die Erfindung betrifft weiterhin die Verwendung wie oben beschrieben, wobei man als Entkopplersubstanzen für proteingebundene Mercaptane, wie Ethanthiol und Methanthiol, physiologische Substanzen mit SH-Gruppen, wie Cystein

Glutathion, Cystein-Peptide, oder Glutaminsäure-Peptide, nichtphysioliogische Substanzen mit SH-Gruppen, wie Dithiothreitol, Fettsäuren oder ihre Salze, wie Ölsäure oder Palmitinsäure oder deren Salze, Substanzen mit SH-Gruppen und Fettsäuregruppen oder ihre Derivate, wie Liponsäure, organische Substanzen mit reduzierenden Eigenschaften, wie Ascorbinsäure, sowie Substanzen, die die Peptid-Thiol-Bindung durch reversible Aufhebung der Tertiärstruktur des Proteins brechen, wie Harnstoff oder Guanidinhydrochlorid, oder Gemische der genannten Substanzen verwendet.

Gemäß einer bevorzugten Ausführungsform verwendet man als Entkopplersubstanzen für das proteingebundene Zytostatikum Methotrexat Salicylsäure, Folsäure, Leucoverin oder Sulfobromphthalein oder deren chemische Derivate, oder Substanzen, die die Protein-Toxin-Bindung durch reversible Aufhebung der Tertiärstruktur des Proteins brechen, wie Harnstoff oder Guanidin·HCl, oder Gemische der genannten Substanzen.

Gegenstand der Erfindung ist weiterhin eine Vorrichtung zur Durchführung des erfindungsgemäßen Verfahrens, die sich durch eine Blutentnahmeeinrichtung mit Leitungen, Ventilen, Pumpen, Blutleckdetektoren und gegebenenfalls Temperiereinrichtungen, einen Plasma-Separationsmodul, gegebenenfalls einen zweiten Plasma-Separationsmodul mit niedrigerer Trenngrenze, eine Entkopplereinrichtung, eine Toxinentfernungseinrichtung, eine Einrichtung zur Vereinigung der Blutbestandteile und eine Blutzuführungseinrichtung für den Patienten auszeichnet.

0102564

Bei einer bevorzugten Ausführungsform der Vorrichtung zur Durchführung des erfindungsgemäßen Verfahrens enthält die Toxinentfernungseinrichtung einen Dialysemodul oder einen Ultrafiltrationsmodul.

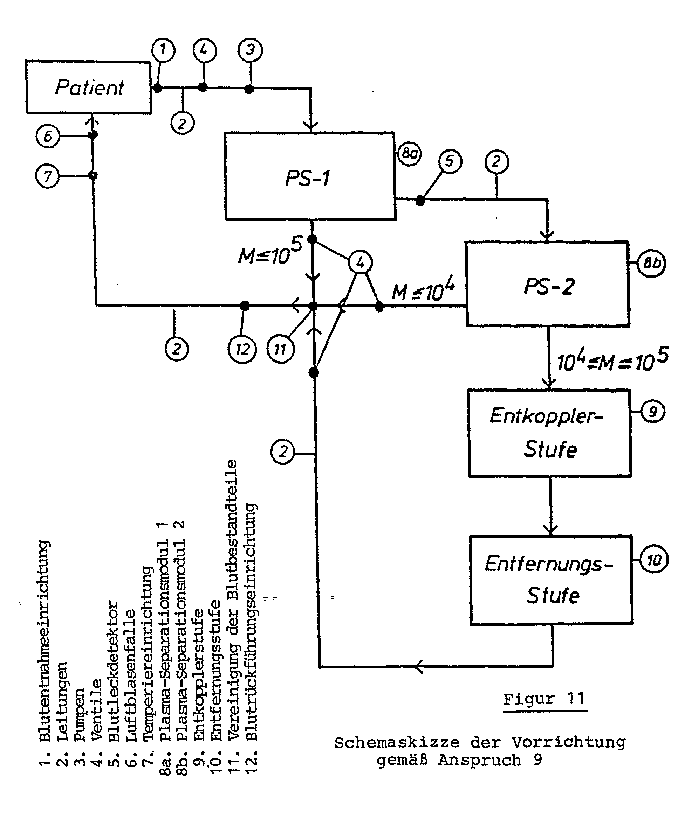

Diese Vorrichtung ist in Figur 11 dargestellt.

Erfindungsgemäß können die beim Leberkoma auftretenden Mercaptane oder Thiole, die zu mehr als 90% an Proteine gebunden sind, insbesondere $CH_3SH$ und $C_2H_5SH$, Phenole, bestimmte Fettsäuren, insbesondere $C_8$-$C_{10}$-Fettsäuren, sowie Gallensäuren entfernt werden. Alle diese Substanzen sind in hohem Maße an Proteine gebunden. Weiterhin können erfindungsgemäß Barbiturate, tricyclische Antidepressiva, Digitalis-Glykoside und Zytostatika, insbesondere Methotrexat, entfernt werden.

Ob ein bestimmtes Toxin an ein Protein oder Lipid gebunden ist, läßt sich relativ leicht nachweisen. Beispielsweise gibt man zu einer Protein- oder Lipidlösung (zum Beispiel käuflich erhältlichem Rinderserumalbumin) eine bestimmte abgewogene Menge des Toxins, läßt das Reaktionsgemisch einige Zeit stehen und bestimmt dann den Gehalt an freiem Toxin mit der Ultrafiltration, Ultrazentrifugation oder mittels chromatographischer Verfahren (GC, GC/MS).

In der folgenden Tabelle I ist die Bindung von Pharmaka an Plasmaprotein dargestellt.

Tabelle I:

Bindung von Pharmaka an Plasmaproteine

| Isoniazid | 100% |
|---|---|
| Coumarin | 99% |
| Phenylbutazon | 98% |
| Diazepam | 96% |
| Imipramin, Chlorpromazin | 90% |
| Digitalis-Glykoside | 90% |
| Diazoxid | 90% |
| Methotrexat | 80% |
| Salicylsäure | 80% |
| Chinin | 80% |
| Chinidin | 60% |
| Chloroquin | 55% |
| Atropin | 55% |

Die in der folgenden Tabelle II aufgeführten Verbindungen sind an Lipide gebunden.

Tabelle II:

Beispiele für lipidgebundene Pharmaka bzw. Toxine

| DDT | lipidgebunden, nicht dialysierbar |
|---|---|
| Paraquat | nicht signifikant dialysierbar lipidgebunden |
| Parathion | nicht signifikant dialysierbar lipidgebunden |
| Ergotamin | nicht signifikant dialysierbar wegen des relativ hohen Molekulargewichts |
| Phenobarbital | nicht signifikant dialysierbar, lipidgebunden |

Toxine können durch Substanzen verdrängt werden, die für die spezifischen Bindungsplätze am Protein oder am Lipid eine größere Affinität aufweisen als das Toxin. Diese Substanzen werden als Entkopplersubstanzen bezeichnet. Als Entkopplersubstanzen werden grundsätzlich physiologische Substanzen bevorzugt, jedoch können gegebenenfalls auch nichtphysiologische Substanzen, die als toxikologisch unbedenklich gelten, Verwendung finden. Diese Substanzen weisen eine höhere spezifische Bindungsaffinität zu den Bindungsplätzen des Toxins auf als das Toxin selbst. Dadurch wird eine Verschiebung des chemischen Gleichgewichts bewirkt, und die Toxine werden freigesetzt. Die freigesetzten Toxine können dann aus dem Blut oder aus den Blutbestandteilen entfernt werden. Entkoppler und Toxin weisen spezifische funktionelle Gruppen auf. Sie stellen aufeinander abgestimmte korrespondierende Substanzpaare dar. Die Entfernung des freigesetzten Toxins erfolgt dann mit konventionellen Methoden (enzymatisch, adsorptiv, durch Membranverfahren etc.).

Im Falle der Entfernung von Methan- und Ethanthiol werden erfindungsgemäß die Entkopplersubstanzen im Überschuß eingesetzt. Als Entkopplersubstanzen kann man physiologische Substanzen mit SH-Gruppen, wie Cystein, Glutathion, Cystein-Peptide, oder Glutaminsäure-Peptide, nichtphysiologische Substanzen mit SH-Gruppen, wie Dithioreitol, Fettsäuren oder ihre Salze, wie Ölsäure oder Palmitinsäure oder deren Salze, Substanzen mit SH-Gruppen und Fettsäuregruppen oder ihre Derivate, wie Liponsäure, organische Substanzen mit reduzierenden Eigenschaften, wie Ascorbinsäure, Substanzen, die die Protein-Thiol-Bindung durch reversible Aufhebung der Tertiärstruktur des Proteins brechen, wie Harnstoff oder Guanidinhydrochlorid, oder Gemische der genannten Substanzen verwenden.

In Vorversuchen läßt sich ermitteln, welche spezifischen Entkoppler für die speziellen Toxine verwendet werden können. Man bindet das Toxin an ein Protein oder Lipid und bestimmt den Gehalt an freiem Toxin. Dann gibt man eine bestimmte Menge an Entkoppler hinzu und bestimmt anschließend erneut den Gehalt an freiem Toxin. Der Fachmann kann daher leicht für ein bestimmtes Toxin einen geeigneten Entkoppler finden.

Toxine mit geringem Siedepunkt werden durch Gaschromatographie mittels der Headspace-Technik bestimmt. Bei Methotrexat wird die freie Toxinmenge durch Ultrafiltration abgetrennt und im Filtrat fotometrisch erfaßt.

In der folgenden Tabelle III sind die bisher ermittelten aufeinander abgestimmten Substanzpaare dargestellt.

<u>Tabelle III:</u>

<u>Toxine und mögliche Substanzen zu ihrer Entkopplung</u>

<u>aus der Proteinbindung</u>

| Toxin | Entkoppler | K-Werte |
|---|---|---|
| Methanthiol | Cystein | |
| Ethanthiol | Glutathion, red. | ca. 100 |
| | Dithiothreitol | |
| Methotrexat | Folsäure | ca. 5 |
| | Leucoverin | ca. 5 |
| | Salicylsäure | ca. 5 |
| | Sulfobromphthalein | ca. 5 |
| | Phenylbutazon | ca. 2 |
| $C_8$-$C_{10}$-Fett-säuren | Kaliumoleat | |
| Phenobarbital | Harnstoff | |
| | Guanidin-HCl | |

$$K = \frac{\text{Mol Entkoppler}}{\text{Mol Toxin}}$$

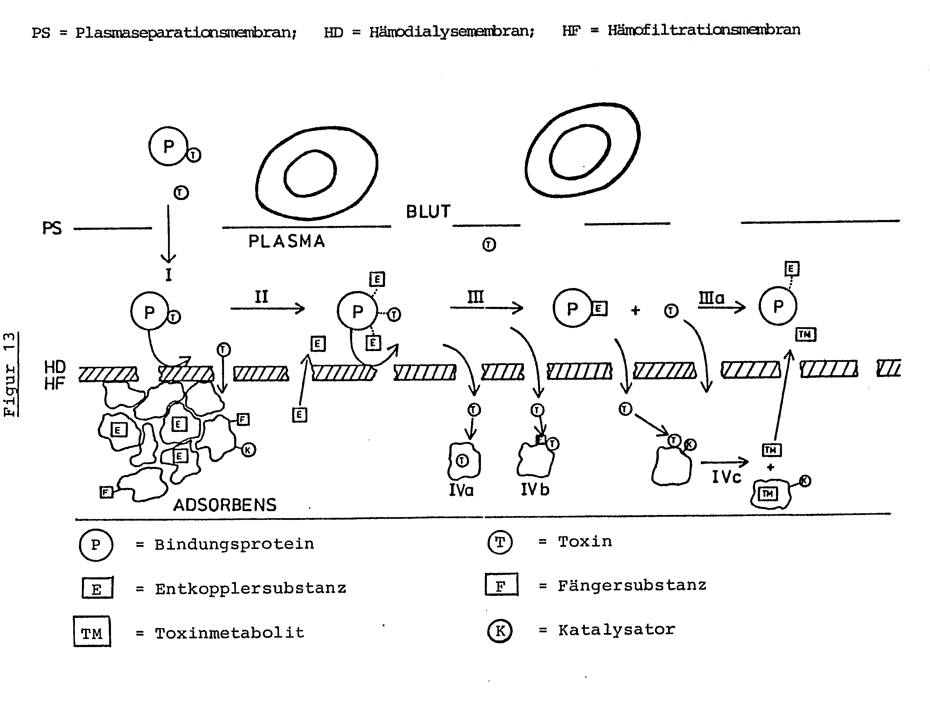

Die Entkopplersubstanzen können entweder in Form von Lösungen zu dem Blut oder der Blutfraktion, welche die gebundenen Toxine enthält, zugegeben werden. Die Entkopplersubstanzen können auch in an Trägersubstanzen, wie Aktivkohle, Aluminiumoxid, Kieselgur und Membranen etc. voradsorbierter Form oder kovalent gebunden vorliegen. Einige der möglichen Prinzipien der Entkopplerzugabe über Membranen zeigen Figur 12 und Figur 13.

Mit Entkopplersubstanzen vorbeladene Trägersubstanzen kann man auf einfache Weise herstellen, indem man zu den Trägersubstanzen eine Lösung mit der Entkopplersubstanz in einem leicht flüchtigen Lösungsmittel in einer bestimmten Konzentration gibt und das Lösungsmittel zum Beispiel im Vakuum abzieht. Kovalente Fixierung ist besonders an den benützten Fraktioniermembranen, aber auch an anionischen Trägern sinnvoll.

Das molare Verhältnis K von Entkoppler und Toxin ist spezifisch für ein Substanzpaar. Werte können Tabelle III entnommen werden.

Die Umsetzung der Entkopplersubstanz mit dem gebunden Toxin erfolgt im allgemeinen in einer Zeit von 10 bis 120 min, bevorzugt von 5 bis 30 min. Man läßt dazu das Reaktionsgemisch bei einer Temperatur von 20 bis 50°C, bevorzugt 35 bis 40°C, in der erfindungsgemäßen Vorrichtung reagieren, wobei die Entkopplereinrichtung je nach Toxin nur einmal oder auch rezirkulierend durchströmt wird.

Nachdem die Entkopplersubstanz das Toxin freigesetzt hat, wird das Toxin aus der Lösung entfernt. Die Entfernung des Toxins aus der Reaktionslösung kann nach an sich bekannten

0102564

Verfahren erfolgen. Beispielsweise kann man die das Toxin enthaltende Lösung einer chemischen Reaktion mit Fängersubstanzen, wie Ascorbinpalmitat, Kaliumoleat oder Lecithin, oder der Adsorption, der Dialyse, der Ultrafiltration, der Affinitätschromatographie oder einer enzymatischen Umwandlung unterwerfen. Beispiele sind in Figur 13 dargestellt.

Zur Entfernung der freigesetzten Toxine wird bevorzugt das Ultrafiltrationsverfahren angewendet.

Die Ultrafiltration ist ein Membran-Trennverfahren. Mit ihr können Lösungen, die aus verschiedenen Komponenten bestehen, nach Molekulargewicht getrennt werden. Dieses Verfahren eignet sich besonders zum Trennen von niedermolekularen Substanzen. Die Ausschlußgrenze, d.h. das Molekulargewicht der Stoffe, die zu ca. 90% zurückgehalten werden, ist durch die gewählte Membran bestimmt. Die treibende Kraft ist eine Druckdifferenz zwischen den beiden Membranseiten. Das gewonnene Ultrafiltrat enthält primär die niedermolekularen Substanzen, d.h. im allgemeinen das Toxin.

Es können jedoch auch geringe Mengen an Protein (ca. 1%) im Filtrat enthalten sein. Bei der Ultrafiltration und Dialyse sind die Verfahrensweisen der einmaligen Passage und der Rezirkulation zu unterscheiden (Figur 14).

Die Entfernung des Toxins kann auch durch Adsorption erfolgen, indem man die Lösung oder Mischung, die das Protein, an welches jetzt der Entkoppler gebunden ist, und das freie Toxin enthält, über den Adsorber leitet, der das Toxin adsorbiert. Beispiele für solche Einheiten sind mit SC-II-Kohle gefüllte Säulen, wie sie beispielsweise für die Bindung von Ethan- und Methanthiol geeignet sind.

Bei der Affinitätschromatographie nützt man z.B. die spezifische Wechselwirkung zwischen einem freien (meist hochmolekularen) Toxin (Antigen) und einem speziell gegen dieses Toxin gerichteten Antikörper, der durch Injektion des Antigens im Blut von Versuchstieren erzeugt wurde und nach
seiner Isolierung beispielsweise in trägerfixierter Form
eingesetzt werden kann (vgl. Figur 15).

Auch durch katalytische Umwandlung des freigesetzten Toxins an einem spezifischen trägerfixierten (immobilisierten) Enzym läßt sich in speziellen Fällen das Toxin unschädlich machen bzw. das Produkt dieser Enzymreaktion
besitzt Eigenschaften, die seine Eliminierung nach einem
der vorbeschriebenen Verfahren gestattet (vgl. Figur 13).

Das vom Toxin befreite Plasma oder die Plasmafraktion wird
entweder zum Patienten zurückgeführt oder mit den anderen
Blutbestandteilen, von denen sie ursprünglich abgetrennt
wurden, wieder vereinigt und dann dem Patienten reinfundiert.

Das erfindungsgemäße Verfahren kann mit Blut oder Blutfraktionen durchgeführt werden. Dem Patienten wird in einem extrakorporalen Kreislauf Blut entnommen, das Blut
wird nach dem erfindungsgemäßen Verfahren behandelt und
danach dem Patienten wieder zugeführt. Bevorzugt wird ein
Verfahren, bei dem das Blut in Blutplasma und Blutkörperchen getrennt und das Blutplasma dann nach dem erfindungsgemäßen Verfahren behandelt wird. Das vom Toxin befreite
Blutplasma wird danach mit den anderen Blutbestandteilen,
d.h. den Blutkörperchen, Fibrinogen etc. vereinigt und
dem Patienten zurückgegeben.

Erfindungsgemäß ist es weiterhin möglich, das abgetrennte
Plasma über Membranen in weitere Fraktionen zu teilen. Beispielsweise kann man durch einen zweistufigen Membranplasmaseparationsprozeß gezielt die Proteinfraktion gewinnen,
in welcher sich das Bindungsprotein befindet (meist die Albuminfraktion).

Solche Blutfraktionen, die das protein- oder lipidgebundene Toxin enthalten, können durch Zentrifugieren, Elektrophorese, Chromatographie, Membrantechniken etc. gewonnen werden. Bevorzugt wendet man Membrantechniken an, beispielsweise die Plasmaseparation.

Die Erfindung betrifft weiterhin eine Vorrichtung zur Durchführung des beanspruchten Verfahrens. Diese Vorrichtung umfaßt eine Blutentnahmeeinrichtung mit Leitungen, Ventilen, Pumpen, Blutleckdetektoren, Luftblasenfalle und gegebenenfalls Temperiereinrichtungen, eine Plasma-Separationsmodul, gegebenenfalls einen zweiten Plasma-Fraktioniermodul, eine Entkopplungsvorrichtung, eine Toxinentfernungseinrichtung, eine Einrichtung zur Vereinigung der Blutbestandteile, eine Blutzuführungseinrichtung etc. (vgl. Figur 11).

Die folgenden Versuche dienen zur besseren Erläuterung der Erfindung.

Verwendete Substanzen:

1. Modelltoxine:

a) Methotrexat (Zytostatikum)     (MTX)
   Molekulargewicht: 454 g/Mol

b) Ethanthiol (endogenes Toxin bei Leberversagen) (ET)
   Molekulargewicht: 62,13 g/Mol

Da Ethanthiol (Siedepunkt: 35$^{\circ}$C) eine stark übelriechende und auch als Gas sehr giftige Substanz ist, wurden alle Versuche, die zur Entwicklung der Meßverfahren notwendig waren, mit Methotrexat durchgeführt.

2. Protein:

a) Rinderserumalbumin (BSA, bovine serum albumin)
b) Humanserumalbumin  (HSA)
c) Rinderfibrinogen    (Fibr)
   (alle von der Firma Behring, Marburg)
d) Humanserumalbumin  (HSA)
   fettsäurefrei
   (Sigma, St. Louis, USA)

Durchgeführte Untersuchungen:

1) Wechselwirkung von Proteinlösungen mit der Quarzküvette

Alle Messungen zur Bestimmung der Protein- sowie der Toxinkonzentrationen wurden mit Hilfe eines Spektralphotometers (HP 8450A Hewlett Packard) durchgeführt. Die photometrische Methode zeichnet sich durch eine kurze Meß-

aus. Dadurch ist ein hoher Probendurchsatz gewährleistet.

Aus der Literatur ist bekannt, daß Proteine an Glasoberflächen adsorbieren und denaturieren können. Adsorbierte Proteine täuschen aber bei photometrischen Messungen höhere Konzentrationen an Proteinen vor, als sie in der Lösung tatsächlich vorliegen.

Es zeigte sich, daß eine Spülung der Küvette mit dem Lösungsmittel PBS (phosphate buffered saline) von ca. 5 Sekunden Dauer ausreichend ist, um eine Veränderung der Basislinie zu vermeiden. Auch Proteinlösungen, die bis zu 1 Minute in der Küvette standen, hinterließen keine Rückstände nach dem Spülvorgang. Die Absorptionsspektren von Proben wiesen zwischen Beginn und Ende der Messung nach 1 Minute keine Unterschiede auf. Die Wellenlänge der Spektren lag zwischen 200 und 500 nm. Die normale Meßzeit beträgt aber nur 10 Sekunden. Es treten also keine Fehler aufgrund von Proteinadsorption auf. Diese Versuche wurden mit BSA, HSA und Fibrinogen durchgeführt.

2) Stabilität von Albumin (BSA) bei Inkubation

Zur Bestimmung des Bindungsverhaltens eines Toxins an ein Protein wird die Lösung bei 37$^{\circ}$C (Körpertemperatur) inkubiert. Daher muß vorher geklärt werden, ob sich das Albumin während der Inkubation verändert. Dazu wurden Proben mit unterschiedlichen BSA-Konzentrationen in Kunststoffröhrchen bei 37$^{\circ}$C auf einem Rollermixer inkubiert. Die BSA-Konzentrationen variierten zwischen 0,1 g BSA/1 PBS und 40 g BSA/1 PBS. Die Konzentration an BSA und die Absorptionsspektren der Proben für die Wellenlänge 200 bis 500 nm wurden wiederholt spektrophotometrisch bestimmt und mit den Ausgangswerten verglichen.

0102564

Dabei zeigte sich, daß Proben mit einer BSA-Konzentration von weniger als 0,6 g/l ca. 5 bis 7 Stunden stabil waren. Proben, deren Konzentration mehr als 1 g/l betrug, wiesen auch nach 24 Stunden noch keine Veränderungen auf. In den folgenden Versuchen sollte der Proteingehalt physiologisch sein. Die HSA-Konzentration im Blut beträgt 40 g/l. Das bedeutet, es können auch Toxine mit geringer Bindungsgeschwindigkeit untersucht werden.

3) Kinetik der Protein-Toxin-Bindung

Zur Bestimmung der Bindungsparameter muß der freie (ungebundene) Anteil des Toxins bestimmt werden. Dazu muß der Bindungsvorgang abgeschlossen sein. Es wurden die Spektren von BSA-MTX-Proben nach 5 Minuten, 1, 2 und 5 Stunden photometrisch gemessen. Die Spektren wiesen nach 5 Minuten Inkubationszeit keine Veränderungen mehr auf. Daher wurde für die Bindungsstudie eine Inkubationszeit zwischen 1 und 2 Stunden festgelegt. In diesem Zeitraum sind auch die Proteinlösungen stabil.

4) Verfahren zur Messung der Protein-Toxin-Bindung

Alle Verfahren zur Messung der Protein-Toxin-Bindung basieren darauf, daß man bekannte Mengen an Toxin und Protein vorgibt und nach der Inkubation die Menge an freiem, ungebundenem Toxin bestimmt. Dazu müssen die Proteinmoleküle (großes Molekulargewicht) von dem Toxin (meist kleines Molekulargewicht) getrennt werden.

Die folgenden Verfahren sind aus der Literatur bekannt:

a) Dialyse
b) Ultrafiltration
c) Ausschlußchromatographie mittels HPLC
d) Ultrazentrifugation

Toxine mit hohem Dampfdruck können auch gaschromatographisch mit Hilfe der Head-Space-Technik bestimmt werden.


## Dialyse

Das Dialyseverfahren (Membranprozeß) basiert auf einem
diffusiven Stofftransport, was eine lange Versuchsdauer
zur Folge hat. Aus diesem Grund wurde dieses Verfahren
nicht weiter verfolgt.


## Ultrafiltration

Die Ultrafiltration eignet sich besonders zum Abtrennen
der Toxine.


## Ultrafiltration als Batch-Verfahren

Es wurden beide Verfahren der Druckerzeugung (Druckluft,
Zentrifugation) erprobt. Dazu wurden die Proben (2 ml)
in die Trennzelle gefüllt und ein Teil (150 µl) abgetrennt. Durch diesen kleinen Probenverlust wird das
Gleichgewicht der Probe nur schwach gestört. Die Konzentrationen der einzelnen Substanzen (Toxin und Protein)
wurden photometrisch mit einem Mehrkomponentenanalyseprogramm ermittelt. Beide Meßverfahren führten zu den
gleichen Ergebnissen. Bevorzugt ist das Verfahren der
Ultrafiltration mit Hilfe der Zentrifugation. Dieses
Verfahren erlaubt einen höheren Probendurchsatz als das
Verfahren mit Druckluft. Die verwendeten Proteinkonzentrationen waren physiologisch (Albumin 40 g/l; Fibrinogen 4 g/l). Aus den bekannten Anfangskonzentrationen und
der gemessenen Konzentration an freiem ungebundenen Toxin lassen sich die Bindungsparameter sowie die Adsorptionsisothermen punktweise ermitteln.


## Ultrafiltration bei konstantem Probenvolumen

Bei diesem Verfahren wird eine Proteinlösung in die Ultrafiltrationszelle eingebracht und mit Druck beaufschlagt. Das anfallende Filtratvolumen wird kontinuierlich durch eine Toxinlösung mit bekannter konstanter Konzentration permanent nachgefüllt. Die Toxinkonzentration im Filtrat wird on-line bestimmt.

Die Vorteile dieses Verfahrens liegen in der hohen Versuchsgeschwindigkeit. In ca. 2 Stunden kann man eine Bindungsstudie durchführen. Die Ergebnisse fallen kontinuierlich und nicht punktweise an.

Nachteilig ist, daß der Filtrierdruck in sehr engen Grenzen gehalten werden muß. Auch das Filtratvolumen muß sehr genau gemessen werden. Ebenso wirken sich Totvolumina (z.B. Schlauchleitungen) stark störend aus. Auch wird die Messung der Toxinkonzentration durch geringe Proteinmengen im Filtrat stark beeinträchtigt.

## Ausschlußchromatographie mittels HPLC

Das Prinzip der Ausschlußchromatographie, einer Trennung nach Molekülgröße (Molekulargewicht), basiert auf dem Siebeffekt. Dabei wird eine mit porösem Trägermaterial gefüllte Säule verwendet. Das Gemisch von Protein und Toxin wird auf die Säule aufgegeben und mit einem Laufmittel eluiert. Die kleineren Toxinmoleküle können in die Poren eindringen und werden somit zurückgehalten. Als erstes werden die Protein aus der Säule eluiert, dann erst die Toxine.

Es wurden zwei Variationen dieses Meßprinzips untersucht: das Laufmittel enthält Toxin, und das Laufmittel enthält Protein und Toxin.

## Ultrazentrifugation

Das Verfahren der analytischen Ultrazentrifugation, bei dem sich das Protein am Boden des Gefäßes niederschlägt, hat den Nachteil, daß es sehr lange dauert; für Vorversuche ist es jedoch durchaus geeignet.

5) Vergleich verschiedener Proteine

Für die Untersuchung der Bindung von Toxinen an Proteine wurden 2 typische Plasmaproteine ausgewählt. Albumin liegt mit der größten Konzentration (40 g/l) im Blut vor. Als weiteres Protein wurde das Fibrinogen (4 g/l) ausgewählt. Die Klasse der Globuline wurde nicht untersucht. Es zeigte sich, daß das Fibrinogen annähernd die gleiche molare Menge von Toxin (ET) wie das Albumin bindet. Unter Berücksichtigung dieses Ergebnisses und der artspezifischen Proteinkonzentration, ergibt sich, daß die an Albumin gebundene Menge an Toxin ca. sechsmal so groß ist wie die von allen anderen Proteinen zusammen. Daher wurden alle folgenden Versuche nur mit Albumin durchgeführt.

6) Bindungsstudien von Toxinen an Protein

Es wurden Bindungsstudien von Toxinen (Methotrexat MTX und Salicylsäure SA) mit Protein (HSA, BSA) durchgeführt. Das freie ungebundene Toxin wurde durch Ultrafiltration gewonnen und die Konzentration photometrisch mit Hilfe der Mehrkomponentenanalyse bestimmt. Die Ausgangsproben enthielten 80 mg Protein und wurden mit PBS auf 2 ml aufgefüllt (physiologische Konzentration). Die Toxinmenge wurde zwischen 100 und 1000 µg variiert. Es zeigten sich deutliche Unterschiede zwischen BSA und HSA.

Die Bindungsfähigkeit von BSA ist größer als die von HSA. Salicylsäure ist stärker gebunden als Methotrexat. Bei Verwendung von BSA liegt MTX zu 10 bis 25% in ungebundener Form vor. Bei Verwendung von HSA schwankt dieser Wert zwischen 30 bis 40%, je nach Ausgangskonzentration.

7) Konkurrenzadsorption von SA und MTX and BSA

Ausgehend davon, daß SA etwas stärker als MTX an BSA gebunden wird, wurde untersucht, ob mit zunehmender SA-Menge die gebundene MTX-Menge abnimmt.

Es zeigte sich, daß in Anwesenheit von SA weniger MTX gebunden wurde. Daraus ergibt sich die Möglichkeit, proteingebundenes MTX durch Zugabe von SA-Lösungen freizusetzen oder in der Bindung zu behindern.

8) Adsorption von Methotrexat an Aktivkohle

Es wurden Adsorptionsversuche von Methotrexat und einer Reihe handelsüblicher Aktivkohlen durchgeführt. Die Proben bestanden aus wäßrigen Lösungen des Toxins ohne Proteinzugabe. Dadurch konnte der Zustand des ungebundenen frei vorliegenden Toxins simuliert werden. Es zeigte sich, daß einige Aktivkohlesorten Methotrexat gut adsorbieren konnten.

10) Bindung von Ethanthiol an BSA

Als zweite Modellsubstanz wurde Ethanthiol ausgewählt. Die Thiolkonzentrationen wurden gaschromatographisch gemessen. Bindungsparameter konnten nicht bestimmt werden, da die Affinität des Thiols zum Albumin so hoch ist, daß die Restkonzentration nicht mehr detektierbar war. Aufgrund der Eichkurven ergab sich, daß innerhalb der Meßgenauigkeit ca. 95% des Ethanthiols an BSA gebunden vorliegt. Die Bindungskinetik ist sehr klein. Die Adsorption des Ethanthiols an BSA bis zur Nachweisgrenze dauert bis zu 6 Stunden.

11) Konkurrenzadsorption von Fettsäuren und Ethanthiol an BSA

Aus der Literatur ist bekannt, daß Fettsäuren eine starke Proteinbindung aufweisen. Proben, die Thiol und Ölsäure (einfach ungesättigte Fettsäure) enthielten, zeigten abnehmende Thioladsorption bei steigender Ölsäurekonzentration. Die Bindung von Ethanthiol an BSA wird durch Zugabe von Ölsäure behindert oder gar blockiert.

12) Adsorption von Ethanthiol an Aktivkohle

Toxine wurden weiterhin adsorptiv entfernt. Dazu wurde eine Reihe handelsüblicher Aktivkohlen und oberflächenaktivierter Aktivkohlen verwendet. Die Kohleeinwaage betrug 0,5 g. Die unbehandelten Kohlen konnten bis zu ca. 15 µl reines Ethanthiol aus wäßriger Phase adsorbieren. Die modifizierten Kohlesorten wiesen zum Teil die doppelte Kapazität auf. Die Restkonzentrationen an Ethanthiol lagen unter denen bei Verwendung von unbehandelten Aktivkohlesorten. Sie scheinen daher als Adsorber bis in den Spurenbereich hinein (absolut geruchsfreie Proben) besonders geeignet zu sein.

Versuchsergebnisse

1) Charakterisierung der Aktivkohle

Zur Entfernung von Ethanthiol (ET) aus einer Proteinlösung wurde die Aktivkohle SCII (Chemviron) ausgewählt. Es handelt sich dabei um eine dampfaktivierte Aktivkohle (AK) auf der Basis von Kokosnußschalen. Um den Druckverlust beim Durchströmen dieser Aktivkohle klein zu halten, wurde eine Korngrößenfraktion zwischen 0,5 und 1 mm ausgewählt.

a) Adsorptionskinetik

Zur Messung der Adsorptionskinetik im Batch-Versuch wurden Proben verwendet, die 30 mg SCII (zuvor in PBS entgast) und 5 ml einer Lösung von 2700 µmol ET/1 PBS ent

hielten. Die Proben wurden bei 37°C auf einen Rollermixer inkubiert. Die Konzentration an ET wurde mit
Hilfe der Headspace-Technik gaschromatographisch bestimmt.

Es zeigte sich, daß nach ca. 30 bis 40 Minuten kein
Ethanthiol mehr nachgewiesen werden konnte. Die Lösung
war geruchsfrei.

b) <u>Adsorptionskapazität im Durchbruchsversuch</u>

Die Durchbruchskurve einer Adsorbersäule gibt Aufschluß
über die Kapazität des Adsorbens und das kinetische Verhalten des Adsorbersystems. Von großem Einfluß sind dabei die Durchflußgeschwindigkeit (Kontaktzeit), die Adsorbensmenge, die Korngröße und die Geometrie der verwendeten Säule.

Die eingesetzte Aktivkohlemenge betrug 500 mg. Das ergibt bei einem Säulendurchmesser von 4,5 mm eine Betthöhe von 4,5 cm. Die Durchflußgeschwindigkeit betrug 6,2
ml/min. Die Durchflußzeit durch den Adsorber (= Kontaktzeit mit der Aktivkohle) lag unter 5 Sekunden. Als Testlösung dienten 1000 ml einer Ethanthiollösung von 2160
$\mu$mol ET/1 PBS (das entspricht der 12- bis 16fachen Konzentration bei endogenem Leberkoma). Die Lösung am Säulenauslauf wurde fraktioniert und die Ethanthiolkonzentration als Funktion der Zeit bestimmt. Die Versuchstemperatur betrug 37°C.

Die Konzentration am Säulenausgang steigt annähernd linear mit der Zeit an. Dies ist sowohl auf die unzureichende Geometrie als auch auf die kurze Kontaktzeit zurückzuführen. Nach ca. 140 Minuten beträgt die Konzentration am Ausgang 50% der Einlaufkonzentration. Eine Mengenbilanz ergibt, daß 71% des eingesetzten Ethanthiols von der Aktivkohle adsorbiert wurden. Für eine Kontaktzeit unter 5 Sekunden ist dies ein gutes Ergebnis. Die mittlere Beladung betrug bei Versuchsende 3000 /umol ET/ g AK (= 9,3 mg ET/g AK). Es wird angestrebt, dieses Ergebnis mit modifizierter AK zu verbessern.

c) Adsorptionsisotherme

Die Adsorptionsisotherme beschreibt das statische Gleichgewicht zwischen der Beladung des Adsorbens (Aktivkohle) und der Adsorptivkonzentration (Ethanthiol in wäßriger Phase) im Batchversuch.

Die Proben wurden bei 37°C über Nacht auf einem Rollermixer inkubiert. Sie enthielten 10 mg SCII und 50 ml PBS. Die Ethanthiolkonzentration wurde zwischen 2700 /umol ET/l PBS und 270 /umol/l variiert. Die Isotherme ist über einen großen Bereich konstant. Das bedeutet, daß die Kohle vollständig beladen ist. Die Grenzbelastung beträgt ca. 0,5 mg ET/mg AK.

2) Spaltung des Ethanthiol-Protein-Komplexes mit Hilfe von "Entkopplern"

Screening-Test der Entkoppler

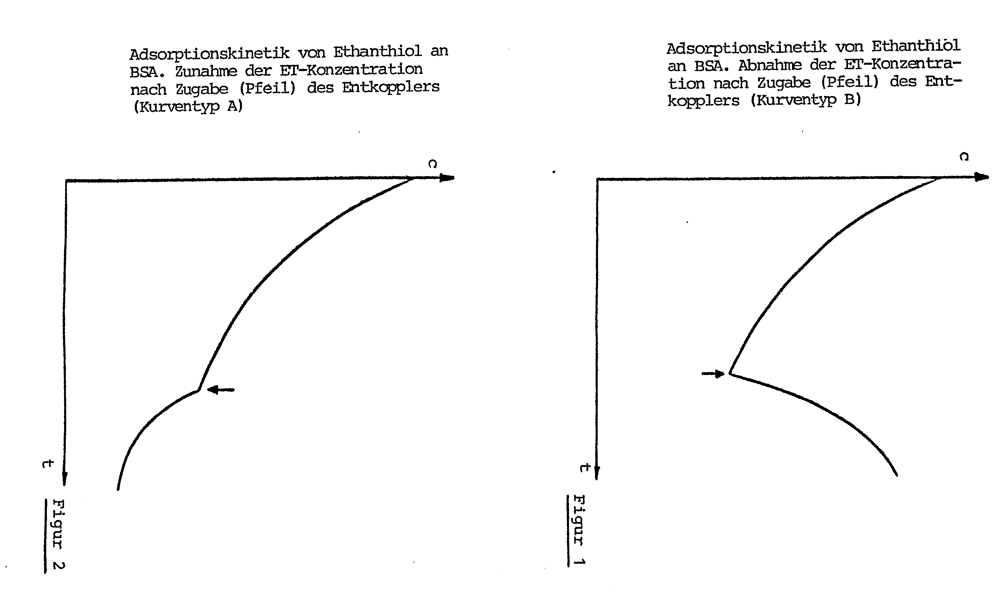

Um Substanzen auf ihre Eignung als Entkoppler zu testen, wurden Proben, die 40 mg BSA (40 g/l physiologische Proteinkonzentration) und 1 ml ET-Lösung (2170 /umol/l) enthielten, für 4 Stunden bei 37°C inkubiert und die ET-Restkonzentration bestimmt. Auf diese Weise kann die Kinetik der Bindung von ET an BSA bestimmt werden. Nach ca. 3 Stunden sind 50% der ET-Menge gebunden. Nach einer Inkubationszeit von ca. 4 Stunden werden 10 bis 20 mg der als Entkoppler zu testenden Substanz zugegeben und die ET-Konzentration gemessen. Es ergaben sich zwei unterschiedliche Funktionstypen (Fig. 1 und 2). Kurventyp A (Fig. 1) zeigt die erwartete Freisetzung von ET nach der Zugabe der Entkopplersubstanz. Die Freisetzung erfolgt erheblich schneller als die Bindung an das BSA.

Substanzen mit einem Verhalten nach Typ A sind:

      a) Ascorbinsäure

      b) Cystein

      c) Dithiothreitol

      d) Glutathion, reduziert

Kurventyp B (Fig. 2) gibt die Abnahme von ET nach Zugabe des Entkopplers wieder. Auch hier ist die Kinetik erheblich größer als für die Bindung an BSA.

Substanzen mit einem Verhalten nach Typ B sind:

      a) Ascorbinpalmitat

      b) Kaliumoleat

      c) Lecithin.

Für die weiteren Versuche wurden Cystein und Glutathion ausgewählt, da beide Substanzen niedermolekular und physiologisch sind.

Variation der Entkoppler-Menge

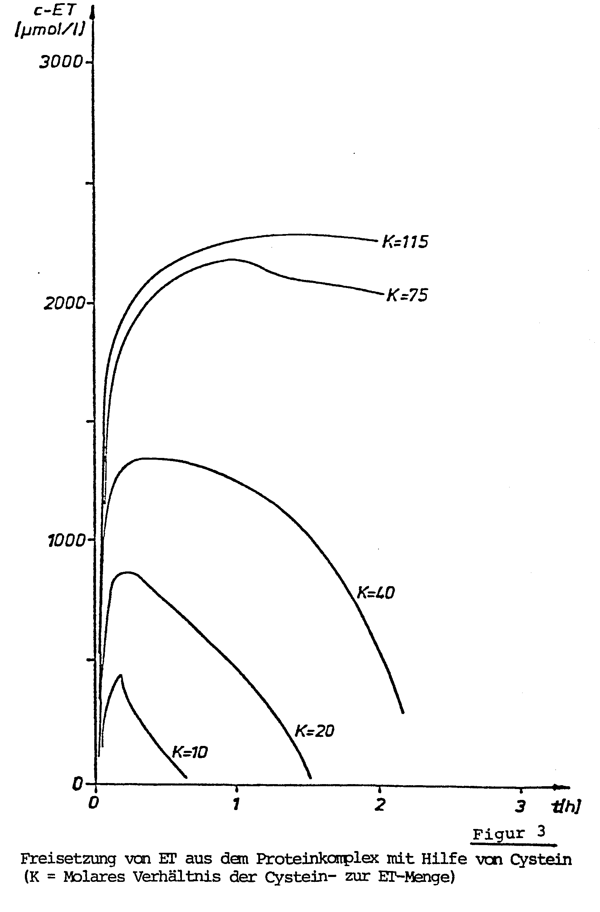

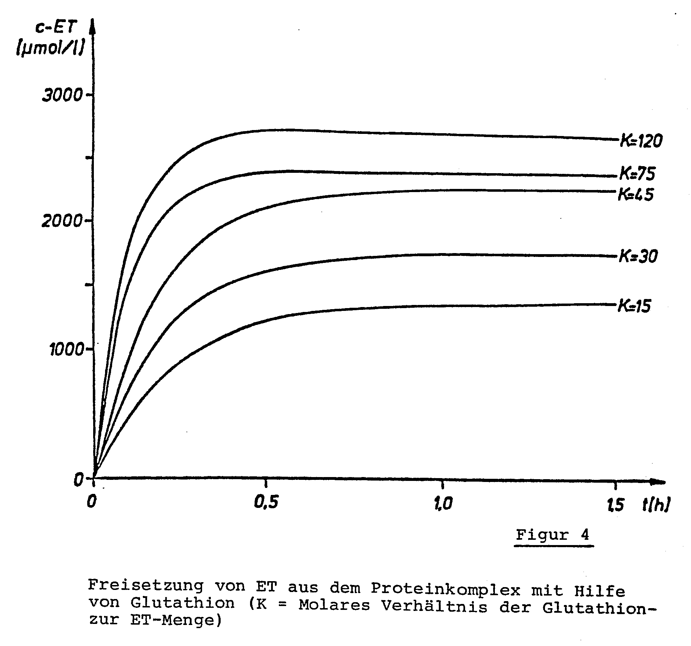

Um die Wirksamkeit der beiden Entkoppler vergleichen zu können, wurden Proben mit 40 mg BSA auf 1 ml ET-Lösung (2170 µmol ET/l PBS) über Nacht bei 37°C inkubiert. Nach dieser Zeit betrug die freie Thiolkonzentration ca. 2 bis 5% der Ausgangskonzentration. Nach der Zugabe unterschiedlicher Entkoppler-Mengen wurde die Freisetzung des ET gaschromatographisch verfolgt. Fig. 3 und 4 zeigen den Verlauf der ET-Konzentration als Funktion der Zeit. Die Zugabe des Entkopplers erfolgte zum Zeitpunkt t = o. Der Parameter K entspricht dem molaren Verhältnis zwischen eingesetzter Entkoppler-Menge und eingesetzter ET-Thiolmenge.

Es zeigt sich in beiden Fällen, daß die freigesetzte Menge an ET und die Kinetik mit zunehmender Entkoppler-Menge ansteigen und ein Freisetzungsgrad von 100% erreicht wird, d.h. die gesamte gebundene ET-Menge kann durch diese beiden Entkoppler wieder freigesetzt werden.

Bei der Verwendung von Cystein zeigt sich für kleine K-Werte eine Abnahme der ET-Konzentration bei längeren Inkubationszeiten (Fig. 3). Dies deutet darauf hin, daß parallel zu dem schnellen Abkopplungs- noch ein langsamer Bindungsprozeß abläuft. Es ist daher für die Anwendung wichtig, daß die Entfernung des freien ET in der Abkopplungs- und der Plateauphase abgeschlossen wird. Die K-Werte für den gleichen Freisetzungsgrad an ET liegen für Cystein höher als für reduziertes Glutathion.

Untersuchung der Adsorption von ET an den Entkoppler

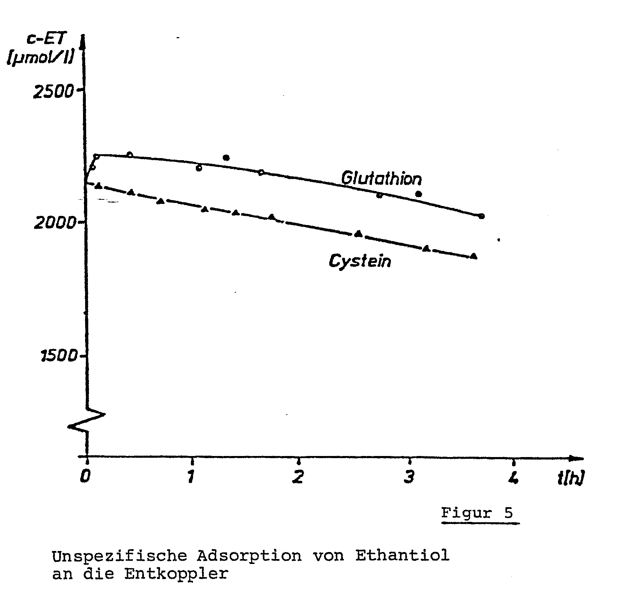

Es wurde beobachtet, daß nach dem Freisetzen des Toxins und der damit verbundenen Zunahme der ET-Konzentration eine Abnahme auftrat. Dieser Effekt könnte auf eine Bindung von ET an den Entkoppler oder auf eine Wechselwirkung mit dem Protein zurückgeführt werden. Um dies zu klären, wurden Proben mit 1 ml ET-Lösung (2170 $\mu$mol ET/ 1 PBS) mit Entkoppler bei 37$^o$C inkubiert. Der K-Wert der Proben betrug 150. Die ET-Konzentration wurde als Funktion der Zeit gemessen (Fig. 5). Die jeweilige ET-Konzentration nach Zugabe des Entkopplers nimmt nur langsam mit der Zeit ab. Sie beträgt nach 3 Stunden noch mehr als 90% der Anfangskonzentration. Vergleicht man diese Kinetik mit der in Fig. 3 und 4, so ist die Bindung von ET an Cystein oder reduziertes Glutathion zu vernachlässigen. Die Abnahme der ET-Konzentration beruht somit bei der Verwendung von Cystein auf einer Wechselwirkung zwischen dem Ethanthiol und dem Protein.

3) Entfernung von proteingebundenem Ethanthiol mit Hilfe von Aktivkohle

Adsorption von ET an AK ohne vorherige Freisetzung

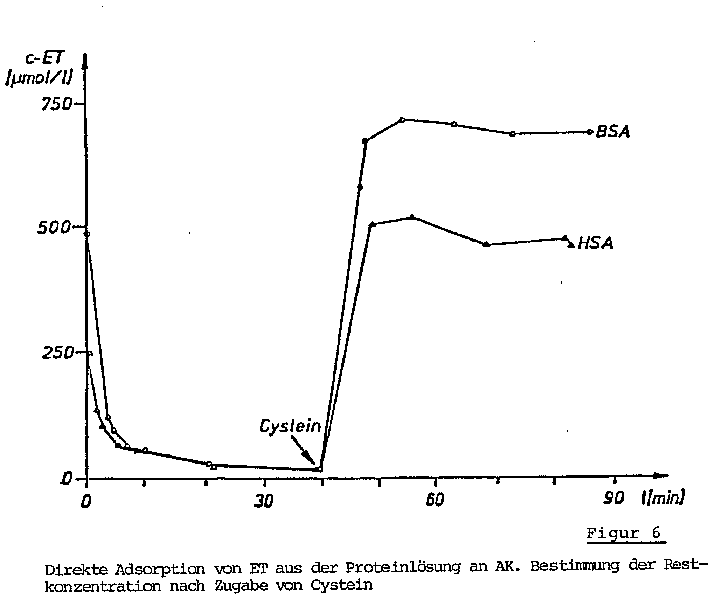

Um die Leistungsfähigkeit der ET-Entfernung nach vorhergehender Abkopplung von Protein beurteilen zu können, wurde die Adsorptionskinetik ohneVerwendung eines Entkopplers untersucht. Dazu wurden im Batchversuch Proben, die 40 mg BSA oder HSA auf 1 ml ET-Lösung (2170 umol ET/ 1 PBS) enthielten, über Nacht auf einem Rollermixer inkubiert. Nach Einstellen des Gleichgewichts wurde am nächsten Tag die Konzentration an ET bestimmt. Sie lag für BSA bei ca. 11% und für HSA bei ca. 21% der Ausgangskonzentration. Zu beiden Proben wurden 30 mg SCII (vorher in PBS entgast) zugegeben und die freie ET-Konzentration fortlaufend gemessen. Nach 45 Minuten wurden die AK entfernt und Cystein zugegeben. Diese Cysteinmenge (K = 110) bewirkte eine totale Freisetzung des noch vorhandenen Ethanthiols. Fig. 6 zeigt den Verlauf der ET-Konzentration. Die Probe, die HSA enthielt, wies eine Abnahme der ET-Menge durch Adsorption von 78% auf, die Probe mit BSA lediglich von ca. 68%. Dieses Ergebnis deckt sich mit der Beobachtung, daß ET stärker an BSA gebunden ist als an HSA. Damit konnte gezeigt werden, daß proteingebundenes ET durch Aktivkohle entfernt werden kann. Die Restkonzentration liegt jedoch relativ hoch, und die Kinetik ist langsam.

## Adsorption von ET an Aktivkohle nach vorheriger Freisetzung

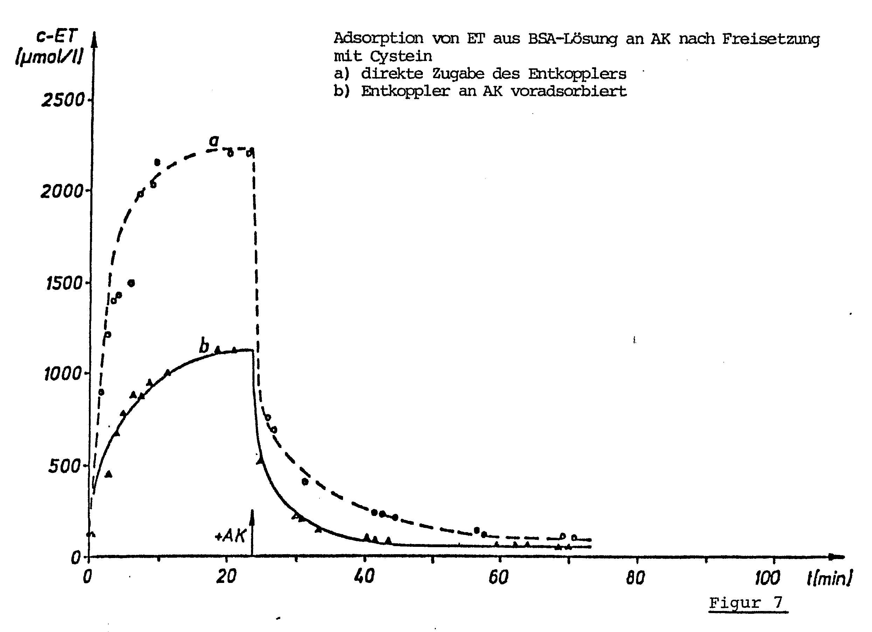

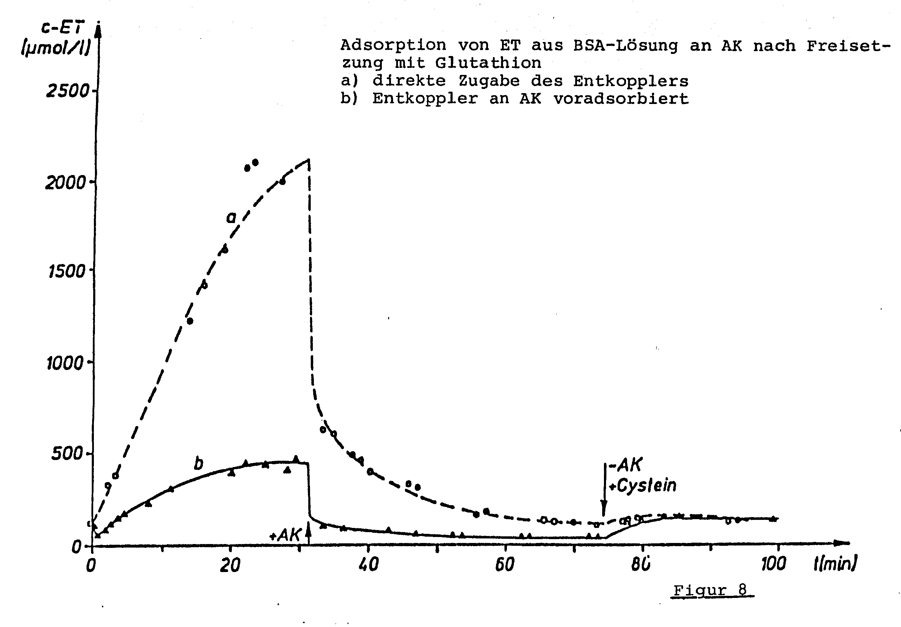

In dieser Versuchsreihe wurde das Zusammenwirken aller bisher betrachteten Parameter im Batch erprobt. 1 ml ET-Lösung (2170 umol ET/1 PBS) wurden für ca. 18 Stunden bei 37°C mit 40 mg BSA inkubiert. Nach dieser Zeit beträgt die ET-Konzentration lediglich 5% des Ausgangswerts (d.h. 95% des ET sind an das BSA gebunden). Nun erfolgte die Zugabe von 150 ul Entkoppler (K-Cystein = 115, Fig. 7, Kurve a; K-Glutathion = 30, Fig. 8, Kurve a). Die Abkopplung des ET wird verfolgt, bis keine Zunahme der ET-Konzentration mehr eintritt. Bei der Verwendung von Cystein erfolgt die Freisetzung schneller als bei Glutathion.

Nun wurden 30 mg SCII (vorher in PBS entgast) zu den Proben hinzugegeben. Die ET-Konzentration nimmt nun rasch ab. Die gesamte Restmenge beträgt nach 45 Minuten nur noch ca. 5% des Anfangswerts. Es wurden also 95% der vorgegebenen Thiolmenge von 30 mg AK entfernt.

In einer weiteren Versuchsreihe wurde untersucht, ob der Entkoppler auch wirksam ist, wenn er zuvor an Aktivkohle adsorbiert wurde. Dazu wurden 30 mg SCII mit den Entkoppler-Lösungen 2 Stunden inkubiert. Diese vorbeladene Kohle wurde dann statt der Entkoppler-Lösung eingesetzt. Fig. 7 und 8 (jeweils Kurve b) zeigen den Abkopplungsvorgang. Die freie ET-Konzentration steigt in beiden Fällen nicht auf den Anfangswert an. Dem Freisetzungsprozeß ist eine Adsorption überlagert. Sie liegt bei AK-Glutathion um ca. 50% tiefer als bei AK-Cystein. Nach Zugabe von 30 mg AK (in PBS entgast) fällt die ET-Konzentration schnell ab. Nach 45 Minuten beträgt die adsorbierte Menge bei Verwendung von Cystein ca. 97%, bei Verwendung von Glutathion ca. 95%.

Die adsorptive Entfernung von proteingebundenem ET nach vorheriger Freisetzung durch einen Entkoppler führt zu erheblich geringeren Restkonzentrationen von ET als die direkte Adsorption. Einen verfahrenstechnischen Vorteil stellt die Desorption der Entkoppler-Substanz von einem Träger dar. Das hat zur Folge, daß keine zusätzliche Pumpe benötigt wird. Der Desorptionsstrom kann durch Diffusionswiderstände (zum Beispiel Coatings) in weiten Grenzen variiert werden. Durch die überlagerte Adsorption bleibt auch die Toxinkonzentration im Plasma relativ niedrig, was eine erhöhte Sicherheit bei vorzeitigem Durchbruch des Adsorbers darstellt.

**4) Entfernung von proteingebundenem ET aus dem Plasma**

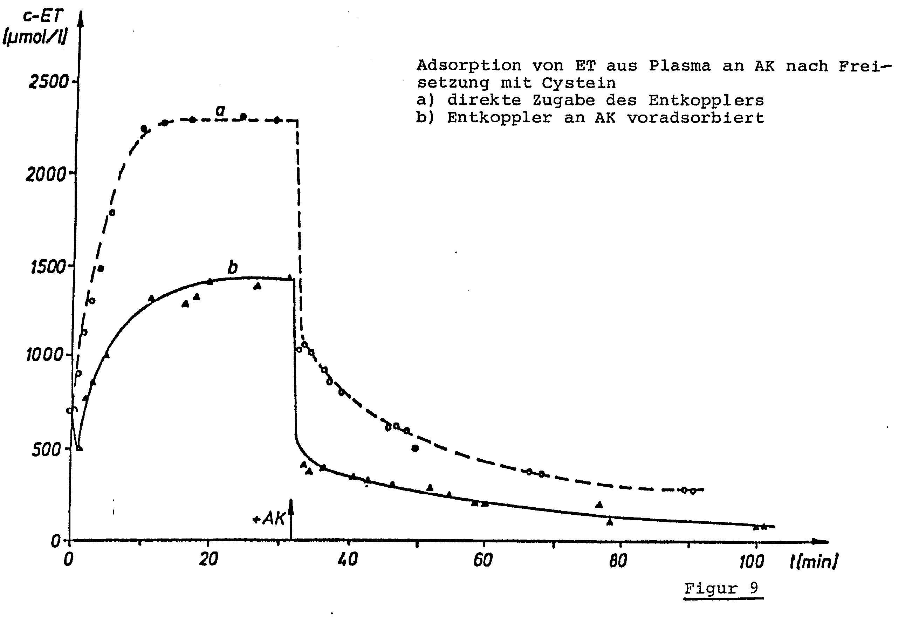

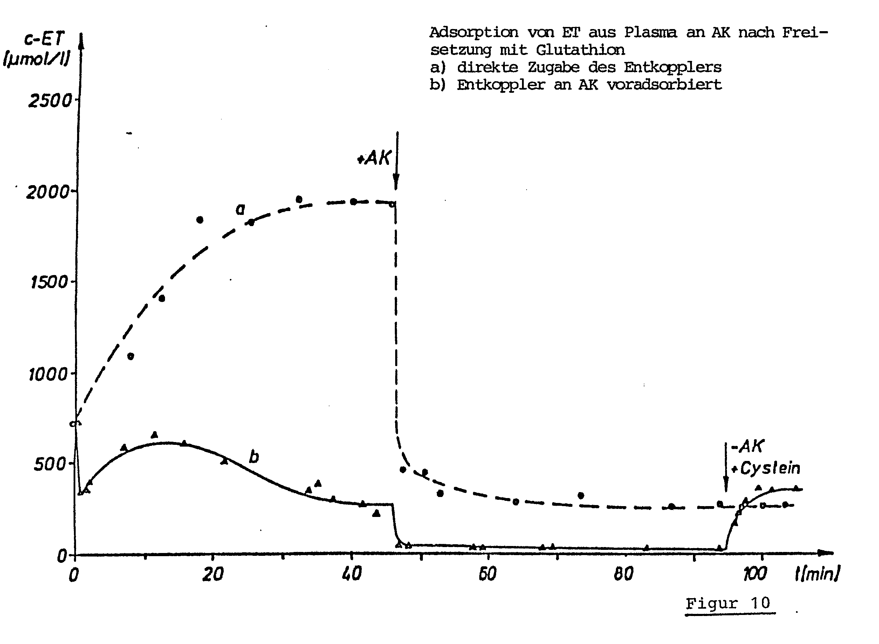

Diese Versuche wurden analog zu den unter 3) beschriebenen durchgeführt. Die Albuminkonzentration des Plasmas betrug 34 g/l. Die Ausgangskonzentration an ET vor der Inkubation betrug 2170 µmol ET/l Plasma. Nach 20 Stunden Inkubation bei 37°C waren lediglich 67% des ET gebunden. Die starke Abweichung im Vergleich zu BSA oder HSA ist auf Konkurrenzadsorption (Belegung des Albumins durch andere Substanzen) und die geringe Albuminkonzentration zurückzuführen. Zu den Plasmaproben wurde als Entkoppler Cystein (Fig. 9) als Lösung oder an AK voradsorbiert oder Glutathion (Fig. 10) hinzugegeben.

Auch hier steigt die freie ET-Konzentration nach Zugabe des Entkopplers stark an. Cystein bewirkt auch hier eine schnellere Freisetzung als Glutathion. Bei Verwendung der voradsorbierten Entkoppler ist die freie Konzentration ebenfalls niedriger als bei der direkten Anwendung. Die freie ET-Konzentration ist auch hier bei Verwendung von voradsorbiertem Glutathion (Fig. 10, Kurve b) wieder am geringsten (analog Fig. 8, Kurve b). Die eigentliche Adsorption erfolgt wieder mit 30 mg AK. Die Restkonzentration an nicht entferntem Ethanthiol (frei und proteingebunden) wurde bei Versuchsende (45 Minuten Adsorption) durch Zugabe von Cystein bestimmt. Dabei zeigt sich, daß die Konzentration bei den mit Glutathion behandelten Proben wieder leicht zunimmt (es hat keine 100%ige Entkopplung stattgefunden).

Die Restkonzentrationen für die einzelnen Versuche im Vergleich zum Ausgangswert zeigt die folgende Tabelle IV.

Tabelle IV: Restkonzentration an ET nach Adsorption an AK mit vorheriger Freisetzung durch Entkoppler

| Entkoppler | Zugabe des Entkopplers | |
| | direkt | voradsorbiert |
|---|---|---|
| Cystein | 15% | 4% |
| Glutathion, red. | 15% | 16% |

Die Restkonzentrationen bei der Entfernung von ET aus Plasma liegen im Vergleich zur Entfernung aus einer BSA-Lösung höher. Der Grund liegt primär in der Konkurrenzadsorption von Plasmabestandteilen an die Aktivkohle.

PATENTANSPRÜCHE

1.    Verfahren zur Entfernung von protein- oder lipidgebundenen Toxinen aus Blut oder Blutfraktionen, dadurch gekennzeichnet, daß man zum dem Blut oder der Blutfraktion Entkopplersubstanzen zugibt, welche die Toxin-Protein-Bindung oder Toxin-Lipid-Bindung lösen, und die freigesetzten Toxine aus dem Blut oder der Blutfraktion entfernt.

2.    Verfahren nach Anspruch 1, dadurch gekennzeichnet, daß man als Entkopplersubstanzen solche Verbindungen verwendet, die eine höhere Affinität zum Bindungsprotein oder Bindungslipid der Toxine besitzen als das Toxin selbst.

3.    Verfahren nach Anspruch 1 oder 2, dadurch gekennzeichnet, daß die Entkopplersubstanzen

im Überschuß eingesetzt werden.

4.    Verfahren nach einem der Ansprüche 1, 2 oder 3, dadurch g e k e n n z e i c h n e t , daß man als Entkopplersubstanzen physiologische oder nichtphysiologische Verbindungen und/oder Gemische von Verbindungen verwendet, die dem zu verdrängenden Toxin entsprechende chemische Strukturelemente, wie funktionelle Gruppen, und physikochemische Eigenschaften, wie Hydrophilie- bzw. Hydrophobizitätsgrad, aufweisen und/oder die in der Lage sind, das jeweilige Toxin chemisch oder physikalisch zu binden (Fängersubstanzen) oder die Tertiärstruktur des Toxinträgers (Protein, Lipid) in reversibler Weise aufzuheben.

5.    Verfahren nach einem der Ansprüche 1, 2, 3 oder 4, dadurch g e k e n n z e i c h n e t , daß man als Entkopplersubstanzen für proteingebundene Mercaptane, wie Ethanthiol und Methanthiol, physiologische Substanzen mit SH-Gruppen, wie Cystein, Glutathion, Cystein-Peptide, oder Glutaminsäure-Peptide, nichtphysiologische Substanzen mit SH-Gruppen, wie Dithiothreitol, Fettsäuren oder ihre Salze, wie Ölsäure oder Palmitinsäure oder deren Salze, Substanzen mit SH-Gruppen und Fettsäuregruppen oder ihre Derivate, wie Liponsäure, organische Substanzen mit reduzierenden Eigenschaften, wie Ascorbinsäure, sowie Substanzen, die die Peptid-Thiol-Bindung durch reversible Aufhebung der Tertiärstruktur des Proteins brechen, wie Harnstoff oder Guanidinhydrochlorid, oder Gemische der genannten Substanzen verwendet.

6.    Verfahren nach einem der Ansprüche 1, 2, 3 oder 4, dadurch g e k e n n z e i c h n e t , daß man als Entkopplersubstanzen für das proteingebundene Zytostatikum Methotrexat Salicylsäure, Folsäure, Leucoverin oder Sulfobromphthalein oder deren chemische Derivate, oder Substan-

0102564

zen, die die Protein-Toxin-Bindung durch reversible Aufhebung oder Tertiärstruktur des Proteins brechen, wie Harnstoff oder Guanidin·HCl, oder Gemische der genannten Substanzen verwendet.

7. Verwendung von physiologischen oder nichtphysiologischen Verbindungen und/oder ihren Gemischen als Entkopplersubstanzen, die dem zu verdrängenden Toxin entsprechende chemische Strukturelemente, wie funktionelle Gruppen, und physikochemische Eigenschaften, wie Hydrophilie- bzw. einen Hydrophobizitätsgrad, aufweisen und/oder die in der Lage sind, das jeweilige Toxin chemisch oder physikalisch zu binden (Fängersubstanzen) oder die Tertiärstruktur des Toxinträgers (Protein, Lipid) in reversibler Weise aufzuheben.

8. Verwendung nach Anspruch 7, dadurch g e k e n n - z e i c h n e t, daß man als Entkopplersubstanzen für proteingebundene Mercaptane, wie Ethanthiol und Methanthiol, physiologische Substanzen mit SH-Gruppen, wie Cystein, Glutathion, Cystein-Peptide, oder Glutaminsäure-Peptide, nichtphysiologische Substanzen mit SH-Gruppen, wie Dithiothreitol, Fettsäuren oder ihre Salze, wie Ölsäure oder Palmitinsäure oder deren Salze, Substanzen mit SH-Gruppen und Fettsäuregruppen oder ihre Derivate, wie Liponsäure, organische Substanzen mit reduzierenden Eigenschaften, wie Ascorbinsäure, sowie Substanzen, die die Peptid-Thiol-Bindung durch reversible Aufhebung der Tertiärstruktur des Proteins brechen, wie Harnstoff oder Guanidinhydrochlorid, oder Gemische der genannten Substanzen verwendet.

9. Verwendung nach einem der Ansprüche 7 oder 8, dadurch g e k e n n z e i c h n e t, daß man als Entkopplersubstanzen für das proteingebundene Zytostatikum Methotrexat Salicylsäure, Folsäure, Leucoverin oder Sulfobromphthalein oder deren chemische Derivate, oder Substanzen, die die Protein-
Toxin-Bindung durch reversible Aufhebung der Tertiärstruktur
des Proteins brechen, wie Harnstoff oder Guanidin· HCl, oder
Gemische der genannten Substanzen verwendet.

Adsorptionskinetik von Ethanthiol an BSA. Abnahme der ET-Konzentration nach Zugabe (Pfeil) des Entkopplers (Kurventyp B)

Figur 1

Adsorptionskinetik von Ethanthiol an BSA. Zunahme der ET-Konzentration nach Zugabe (Pfeil) des Entkopplers (Kurventyp A)

Figur 2

2/14

0102564

Figur 3

Freisetzung von ET aus dem Proteinkomplex mit Hilfe von Cystein
(K = Molares Verhältnis der Cystein- zur ET-Menge)

Figur 4

Freisetzung von ET aus dem Proteinkomplex mit Hilfe
von Glutathion (K = Molares Verhältnis der Glutathionzur ET-Menge)

4|14

Figur 5

Unspezifische Adsorption von Ethantiol
an die Entkoppler

Figur 6

Direkte Adsorption von ET aus der Proteinlösung an AK. Bestimmung der Restkonzentration nach Zugabe von Cystein

Figur 7

Adsorption von ET aus BSA-Lösung an AK nach Freisetzung mit Glutathion
a) direkte Zugabe des Entkopplers
b) Entkoppler an AK voradsorbiert

Figur 8

Figur 9

Figur 10

10/14

Figur 11

Schemaskizze der Vorrichtung
gemäß Anspruch 9

**Figur 12**

Möglichkeiten der Entkopplerzugabe (E) gemäß Anspruch 10 (Entkopplerstufen)

a) direkte Zugabe über eine Mischstrecke;  b) Desorption von einem vorbelegten Adsorbens (A)

c) Desorption aus einem vorbelegten Membransystem;   d) Entkoppler kovalent an einen Träger(z.B.Memtran)fixiert

e) Zugabe aus einem Flüssigkeitsreservoir über eine Membran (Dialyse)

PS = Plasmaseparationsmembran;    HD = Hämodialysemembran;    HF = Hämofiltrationsmembran

12/14

Figur 13

BLUT

PLASMA

PS

I

II

III

IIIa

HD
HF

IVa    IVb    IVc

ADSORBENS

| (P) | = Bindungsprotein | (T) | = Toxin |
| [E] | = Entkopplersubstanz | [F] | = Fängersubstanz |
| [TM] | = Toxinmetabolit | (K) | = Katalysator |

a)     b)     c)     d)     e)

A = Adsorbens     F = Fängersubstanz     T = Träger

Möglichkeiten der Abtrennung des freigesetzten Toxins gemäß Anspruch 8     **Figur 14**
Entfernungsstufen)
a) durch direkte Adsorption;     b) durch physikalische oder chemische Bindung an voradsorbierte Fängersubstanzen;
c) durch physikalische oder chemische Bindung an kovalent gebundene Fängersubstanzen;     d) durch Ultrafiltration;
e) durch Dialyse

○ Albumin    ● Toxin

▨ Verdränger    ▢ Antikörper

Kombinierte Anwendung immunologischer und affinitätschromatographischer Prinzipien
zur Entfernung protein(oder auch lipid-)gebundener (hochmolekularer) Toxine

PLASMA          MEMBRAN
                (Träger)

① ⑩

Weg 1: Bindung des Protein-Toxin-Komplexes an den trägerfixierten Antikörper gegen dieses Toxin unter Ausbildung eines Protein(Lipid)-Toxin-Antikör-
per-Membran-Komplexes; danach Freisetzung des Protein(Albumin) durch Zugabe
einer Verdrängersubstanz (Weg 2). Weg 1a: Wechselwirkung zwischen ungebundenem Protein-Toxin-Komplex und spezifischem Antikörper in Gegenwart der
Verdrängersubstanz, ebenfalls unter Ausbildung eines Antigen(=Toxin)-Anti-
körper-Komplexes und Freisetzung des ursprünglichen Komplexpartners(Protein
bsw.Lipid)

Figur 15